# EUROPEAN PATENT APPLICATION

(11) **EP 2 260 857 A1**
(43) Date of publication of application: **15.12.2010**
(21) Application number: 09290437.4
(22) Date of filing: 11.06.2009
(51) Int. Cl.: A61K 38/00

(54) **Novel applications of HIP/PAP or derivatives thereof**

(71) Applicant: Alfact Innovation, 75001 Paris (FR)
(72) Inventor: Faivre, Jamila, 75016 Paris (FR); Amouyal, Gilles, 75016 Paris (FR); Amouyal, Paul, 92310 Sevres (FR); Brechot, Christian, F-75015 Paris (FR); Gressens, Pierre, F-95470 Saint-Witz (FR); Rougier, Elodie, F-87600 CHERONNAC (FR)
(74) Representative: Catherine, Alain

(57) **Abstract**

The invention consists of the use of the HIP/PAP protein or a protein derivative thereof for preventing or treating a disease selected from the group consisting of a neonatal brain injury, an adult brain injury caused by a brain hypoxia and a disease involving a defect in the production or in the phosphorylation of Gap-43.

## Description

### FIELD OF THE INVENTION

This invention relates to the field of neurology.

This invention relates to the field of neonatal neurology.

More precisely, the present invention relates to the field of the medical treatment of neurodegenerative diseases, and especially the medical treatment of neurodegenerative diseases caused by hypoxia or of brain injury caused by various traumas, including cerebrovascular accident and trauma of the spinal cord.

The instant invention relates more particularly to the field of neonatal brain injury.

### BACKGROUND OF THE INVENTION

Neonatal brain injury constitutes a specific field of medical investigations, since processes involved in neonatal neuronal injury are distinct from those leading to neuronal injury in adult patients.

In spite of improved obstetric and neonatal care, two groups of newborn infants are still at risk of developing perinatal brain injury with permanent neurological impairment, namely the preterm infant with predominantly white-mater damage and the term asphyxiated infant with hypoxic-ischemic brain injury. In addition to hypoxia-ischemia, an enhanced release of glutamate leading to excitotoxic cell death and the production of reactive oxygen species (ROS) are important in the brain injury process in both these groups.

Neonatal asphyxia, also known as hypoxia-ischemia (HI), is a condition arising from the inadequate intake of oxygen in an infant during preterm development, labour, delivery, or the immediate postnatal period. Neonatal asphyxia remains a major cause of chronic neurological morbidity and acute mortality in the newborn and commonly leads to hypoxic-ischemic encephalopathy. The existing studies have shown that neonatal hypoxia for a time as short as six minutes can lead to permanent neurological damage. About 14.6% of all deaths at birth are caused by neonatal hypoxia. In the western world, about 0.9% of newborns suffer from neonatal hypoxia. About 15-20% die, and among the survivors, 25% are severely handicapped due to long-term complications such as mental retardation, cerebral palsy, spasticity, learning difficulties or epilepsy. Furthermore, it is increasingly recognized that children with rather mild hypoxia, who seem initially to recover without complications, have behavioural problems in childhood, which can be tracked back to this neonatal insult.

Generally, the initiation and development of injury to the neonatal brain are complex processes, with multiple contributing mechanisms and pathways resulting in both early and delayed injury. Studies have focused on various aspects of these processes, including oxidative stress, inflammation and excitotoxicity, which are widely different in the mature or immature brain (Gonzalez et al., 2008, Pharmacology and Therapeutics, Vol. 120: 43-53).

Currently, there are no approved therapies for the brain damage associated with neonatal HI beyond the supportive therapies and interventions provided as part of intensive care. However, there is a lot of promising avenues of research to improve intervention and discover new strategies in the wake of the therapies that have been tested for clinical applications, and failed. Examples of failed therapies include supplemental glucose during resuscitation, hyperventilation, midazolam, barbiturates, dexamethasone and other glucocorticoids, calcium-channel blockers, magnesium sulfate, indomethacin, superoxide dismutase/catalase conjugated to polyethylene glycol, MK-801, detromethorphan and caspase inhibitors. The management of neonatal cerebral HI includes supportive care to provide adequate ventilation, meticulous fluid management, avoidance of hypotension and hypoglycemia, and treatment of seizures. Indeed, it has been hypothesized that improving the manner in which infants are resuscitated post-HI may be a crucial first step forward for improving outcome and reducing disability. The concentration of oxygen used during resuscitation may affect brain damage after HI. Hyperbaric oxygen has been tried in neonates with HI and in rodent models of focal ischemic stroke with some success. Further studies are required to clarify the role of oxygen in neonates with stroke, who are also at risk for 'oxygen free radical disease.

Beyond improving intensive care, many studies have been performed to identify new treatment options and potential interventions to ameliorate the process of ongoing brain injury. Briefly, various strategies have been explored, including testing antioxidants, anti-inflammatory agents, cell death inhibitors, growth factors, stem cell therapy or hypothermia. A variety of anti-inflammatory compounds have been investigated. Several interventions, such as allopurinol/oxypurinol, desferoxamine and melatonin, attempt to reduce HI-induced cerebral damage resulting from free radicals have failed to raise clinical interest because of their side-effect profiles and some failed trials. Recent promising therapies include erythropoietin and N-acetylcysteine. Erythropoietin is an antioxidant that possesses antiapoptotic, antinitrosative and angiogenic properties. Erythropoietin has also been reported to promote neuroprotection and neurogenesis through the alteration of cell-fate decisions, and to improve functional outcome after stroke in immature rats. N-acetylcysteine, a well-known antioxidant and free-radical-scavenging agent, can cross the placenta and the blood brain barrier (BBB) and has a good safety profile during pregnancy. *N-*acetylcysteine has been shown to reduce oxidative stress, restore intracellular glutathione levels, scavenge oxygen free radicals, improve cellular redox potential and reduce apoptosis and inflammation of the neonatal brain, in addition to the improved cardio-renal recovery observed after asphyxia. Hydrogen therapy might also represent a treatment strategy to explore. Additionally, attempts to reduce brain injury in neonatal hypoxic-ischemic rats by intra-cerebroventicular injection of neural stem/progenitor cells together with chondroitinase ABC have been made (Sato et al., 2008, Reprod Sci., Vol. 15(n6) : 613-620).

There is a need in the art for identifying further compounds that may effectively treat neonatal brain injury.

### SUMMARY OF THE INVENTION

The instant invention provides further compounds for treating neonatal brain injury.

The instant invention relates to the use of the HIP/PAP protein or a protein derivative thereof for preventing or treating a brain injury caused by a brain hypoxia.

It notably relates to the use of the HIP/PAP protein or a protein derivative thereof for preventing or treating a brain injury consisting of a neonatal brain injury.

It notably relates to the use of the HIP/PAP protein or a protein derivative thereof for preventing or treating a brain injury consisting of a cerebrovascular accident or a traumatic brain injury.

It also pertains to the use of the HIP/PAP protein or a protein derivative thereof for in vitro or *in* vivo modulating, enhancing or improving neuronal growth or plasticity mediated by Gap-43, a neuronal growth-related protein. This invention thus also concerns the use of the HIP/PAP protein or a protein derivative thereof for preventing or treating a disorder or a disease involving a deregulation of, or a defect in, Gap-43 production or a deregulation of, or a defect in, Gap-43 phosphorylation.

### DETAILED DESCRIPTION OF THE INVENTION

It has been found herein that the HIP/PAP protein or a protein derivative thereof is able to protect against neuronal cell death occurring in neonatal brain injury.

One is reminded herein that the HIP/PAP protein was previously known in the art as a mitogenic factor for hepatocytes. HIP/PAP (Reg IIIa) gene was also previously known for being up-regulated in a mouse experimental model of an inflammatory brain disease, namely autoimmune encephalomyelitis (EAE), and HIP/PAP protein was shown to potentially exert a preventive effect on mouse EAE. These preliminary data had led previous authors to expect a beneficial effect of Reg III protein for treating a very large number of inflammatory or neurological diseases of the adult patients, including multiple sclerosis, optic neuritis, neuromyelytis optica, adrenoleukodistrophy, adrenomyeloneuropathy, spinal cord injury, amyotrophic lateral sclerosis, inflammatory bowel disease or also sepsis (See Unites States patent application n° US 2005/02 77593). It was also shown in the art that there exists a correlation between allelic polymorphisms in the genomic sequence of the HIP/PAP gene and the occurrence of multiple sclerosis (See PCT application n° WO 2007/071437). Finally, it was als o shown in the art that Reg family members would be mediators among injured neurons and glial cells and would potentially play a role during nerve regeneration (See Namikawa et al., 2005, Biochem Biophys Res Commun, Vol. 332 (n°1): 126-134).

It has been found according to the invention that the HIP/PAP protein, or a protein derivative thereof, possesses neuroprotective properties during neonatal brain injury, thus during a brain damage occurring in the early development which brain damage is completely distinct from the brain injury happening to the developed brain of the adults.

More precisely, it has been found herein that HIP/PAP possesses *in vitro* and *in vivo* neuroprotective effects in experimental models of neonatal brain injury.

The present invention relates to the use of the HIP/PAP protein or a protein derivative thereof for preventing or treating a disease selected from the group consisting of a neonatal brain injury and an adult brain injury caused by a brain hypoxia.

It thus relates to the use of the HIP/PAP protein or a protein derivative thereof for manufacturing a medicament for preventing or treating a disease selected from the group consisting of a neonatal brain injury and an adult brain injury caused by a brain hypoxia.

It also relates to a method of preventing or treating a disease selected from the group consisting of a neonatal brain injury and an adult brain injury caused by a brain hypoxia, wherein the said method comprises a step of administering, to the patient in need thereof, a suitable amount of a HIP/PAP protein or of a protein derivative thereof.

This invention encompasses the use of the HIP/PAP protein or a protein derivative thereof for preventing or treating neonatal brain injury.

It thus pertains to the use of the HIP/PAP protein or a protein derivative thereof for manufacturing a medicament for preventing or treating neonatal brain injury.

It also relates to a method for preventing or treating a neonatal brain injury, wherein the said method comprises a step of administering, to the patient in need thereof, a suitable amount of a HIP/PAP protein or of a protein derivative thereof.

In most embodiments of the preventive or therapeutic methods described in the present specification, the said HIP/PAP protein or the said protein derivative thereof is combined with one or more pharmaceutically acceptable excipients, to form a pharmaceutical composition.

This invention provides novel clues to the neuronal protection against any brain injury/trauma/degeneration by providing new protective compositions.

By "HIP/PAP protein", it is intended herein a protein comprising the amino acid sequence of SEQ ID N°1. Specific embodiments of a HIP/PAP protein encompass proteins comprising an amino acid sequence selected from the group consisting of SEQ ID N°2 and SEQ ID N°3. The HIP/ PAP protein of SEQ ID N°3 consists of the HIP/PAP protein that is more specifically illustrated in the examples herein. As illustrated in the examples herein, the HIP/PAP protein of SEQ ID N°3 may be recombinantly produced in *E. coli.* The HIP/PAP protein of SEQ ID N°3 comprises a N-terminal propeptide portion of 12 amino acids that is cleaved *in vivo* for releasing the biologically active portion of the said HIP/PAP protein. The said biologically active portion of the said HIP/PAP protein is located from the Isoleucine residue in position 13 to the aspartic acid residue located in position 150 of SEQ ID N°3. Similarly; the said biologically active portion of the said HIP/PAP protein is located from the tryptophan residue in position 12 to the aspartic acid residue located in position 149 of SEQ ID N°2.

Preferably, any embodiment of a HIP/PAP protein, as well as any embodiment of a derivative thereof, consists of a recombinant protein, e.g. a protein that is recombinantly produced in bacterial or animal cells, including insect cells and mammal cells.

The inventors have shown that HIP/PAP protein significantly reduces neuron apoptosis in a neonatal mouse model of excitotoxic brain lesions.

The invention also provides therapeutic tools against the oxidative stress, which is a major factor of neuronal abnormalities and degeneration, through the antioxidant power of HIP/PAP, as further detailed below. Moreover, and interestingly, the effect of HIP/PAP protein is also observed when it is administered at the periphery, instead of being directly delivered to the CNS. Finally, protective effects of HIP/PAP protein encompass prevention of neuronal death and induction of neuronal plasticity.

As it is shown in the examples herein, a HIP/PAP protein prevents *in vitro* the N-methyl-D-aspartate (NMDA)-induced neuronal cell death in primary mice neuronal cultures. It is also shown herein that a HIP/PAP protein rescues *in vitro* the N-methyl-D-aspartate (NMDA)-induced neuronal cell death in primary mice neuronal cultures.

Also, it is shown according to the invention that a HIP/PAP protein prevents *in vivo* the excitotoxic lesions caused by the glutamanergic analogue ibotenate in the developing mouse brain.

Additionally, the results presented in the examples disclose that a HIP/PAP protein possesses an *in vivo* rescuing effect on the excitotoxic lesions caused by the glutamanergic analogue ibotenate in the developing mouse brain.

Still further, it has been found according to the invention that the preventive and curative *in vivo* effects of the HIP/PAP protein on neonatal brain injury might be the result of pleiotropic activities of HIP/PAP, i.e. of the involvement of HIP/PAP in a plurality of neuronal rescuing physiological pathways.

As an illustration of the pleiotropic activities of HIP/PAP in the prevention or the treatment of neonatal brain injury, it is also shown herein that HIP/PAP effects neuroprotection against neonatal neuronal cell death induced by a reactive oxygen species like hydrogen peroxide (H₂O₂).

As a further illustration of the pleiotropic activities of HIP/PAP in the prevention or the treatment of neonatal brain injury, it is also shown herein that, in the *in vitro* and *in vivo* models of neonatal brain injury, HIP/PAP induces changes in the production, phosphorylation and distribution of GAP-43 or phosphorylated GAP-43, which is a protein known in the art to be involved in axonal growth and neuronal plasticity.

Notably, HIP/PAP induces a fascicular distribution of GAP-43-labelled neuronal processes.

Also, HIP/PAP induces an increased expression of the phosphorylated form of the GAP-43 protein, i.e. an increased expression of the form of GAP-43 which is phosphorylated at the serine residue located at position 41 (S41) of the GAP-43 amino acid sequence. HIP/PAP rather induces a decrease in the total cellular amount of GAP-43 while, at the same time, it induces an increase in the phospho(S41)-GAP-43.

Without wishing to be bound by any particular theory, the inventors believe that the HIP/PAP biological effects on GAP-43 are important for preventing or treating a brain injury caused by a brain hypoxia, and especially for preventing or treating a neonatal brain injury. In this context, the inventors believe that the effects of HIP/PAP on GAP-43 production and metabolism, by enhancing neuronal plasticity, stimulates novel connexions between living neuronal cells, so that it moderates the consequences of the death or of the apoptosis of certain neuronal cells.

The above experimental results support the usefulness of a HIP/PAP protein, both *in vitro* and *in vivo,* for modulating, enhancing or improving neuronal growth or neuronal plasticity mediated by Gap-43.

As used herein, a brain hypoxia encompasses situations wherein a brain injury is caused by a reduced oxygen supply to the brain, and especially to neuronal cells, and wherein brain hypoxia causes neuronal cell death or apoptosis. Brain hypoxia is considered as occurring when blood vessels are obstructed and/or when there occurs any other cause of oxygen depletion in the brain tissues, especially asphyxia.

As used herein, a "neonatal" brain injury encompasses any injury that is caused to the brain of foetus (also termed foetal brain injury), pre-term neonates, term and post-term neonates, as well as children being less than one year old. Thus, neonatal brain injury encompasses what is conventionally termed « perinatal » brain injury which occurs immediately before and after delivery, as well as what is conventionally termed neonatal brain injury, which occurs during the perinatal period up through four weeks of age. Neonatal brain injury includes stroke, birth trauma, metabolic or genetic disorders, status epilepticus, and a variety of asphyxial events resulting in hypoxia and ischemia (HI). HI often leads to periventricular white matter injury in premature infants, while term infants develop cortical/subcortical lesions. Generally, it is admitted in the art that neonatal brain injury is recognised on the basis of a unique form of encephalopathy that evolves from lethargy to hyperexcitability to stupor during the first three days of life. A review of the available methods of diagnosing neonatal brain injury is disclosed by Ferriero, to which the one skilled in the art may refer, the entire content of which is herein enclosed (Ferriero, 204, New England Journal of Medicine, Vol. 351 (n° 19) : 1985-1995)

In foetal life, neonatal brain injury may be caused (i) by hypoxic-ischemic disturbances due to hypoperfusion induced by low arterial partial pressure of oxygen or (ii) by severe hypoxemia that leads to myocardial dysfunction with subsequent cerebral hypoperfusion leading to neuronal ischemia.

Neonatal brain injury encompasses foetal hypoxemia that is usually the result of placental insufficiency.

Neonatal brain injury also encompasses perinatal hypoxemia, which is a post-natal hypoxemia resulting generally from either respiratory or cardiac insufficiency, alone or in combination.

Neonatal brain injury encompasses foetal ischemia.

Neonatal brain injury encompasses perinatal ischemia, which is a post-natal ischemia , which may be due to a severe cardiac contractile dysfunction that leads to cerebral hypoperfusion and loss of cerebrovascular regulation. Circulatory insufficiency may result from severe prenatal or post-natal haemorrhage or neonatal sepsis.

Neonatal brain injury also encompasses the injury which is caused by pressure-passive cerebral circulation, where autoregulation of cerebral circulation is impaired and hypoxemia is induced.

Neonatal brain injury also encompasses neonatal hypoxia that is due to an underperfusion that affects the depths of the sulci and generates an increased susceptibility to hypotensive insults.

Indeed, neonatal injury encompasses excess release or excitatory neurotransmitters during neonatal brain hypoxia.

According to the present invention, a HIP/PAP protein or a protein derivative thereof may be used for preventing or treating a neonatal brain injury with a grading classification ranging from stage 1 to stage 3, according to the conventional grading system of Sarnat used for classifying hypoxic-ischemic encephalopathy (HIE).

Stage 1 of the Sarnat grading system relies on the finding of an early syndrome characterized by hyperalertness and sympathetic activation, excessive reaction to stimuli, weak suck with normal tone, and electroencephalogram (EEG) findings. This stage lasts less than 24 hours, and patients who remain in stage 1 have normal neurologic outcomes.

Patients progressing to stage 2 of the Sarnat grading system have mild hypotonia, are lethargic or obtunded (e.g., have a delayed and incomplete response to sensory stimuli), clinical seizures, parasympathetic activation with myosis (even in dim light), slowed heart rate ( <120 bpm), increased peristalsis, and copious secretions. Early on, EEG shows relatively low-voltage amplitude (<25 µV in the slow theta and delta range). On the second day, the EEG demonstrates a bursting pattern during wakefulness or obtundation (which worsens during sleep) and multifocal, low-frequency (1- to 1.5-Hz) EEG seizures with a central or temporal predominance. If the EEG recovers within 5 days, it becomes completely normal again. If the recovery takes more than 5 days, low-amplitude slowing is noted. Stage 2 lasts 2-14 days. Clinical and EEG recovery within 5 days is associated with a good prognosis. Periodic EEG may indicate a poor prognosis if interburst intervals are totally isoelectric or if the bursting frequency is less than every 6 seconds, with a bursting pattern (every 3-6 seconds) lasting more than 7 days.

Stage 3 of the Sarnat grading system is characterized by stupor with response to only strong stimuli, with withdrawal or decerebrate posturing, severe hypotonia (e.g. flaccidity) and suppression of deep tendon reflexes, primitive (e.g. Moro, tonic neck, suck) reflexes, and brainstem (e.g. corneal, oculocephalic, gag) reflexes. Clinical seizures are less frequent in stage 3 than in stage 2. The patient has generalized sympathetic or parasympathetic autonomic dysfunction with abnormal respiration and small or midposition pupils that are poorly reactive to light. EEG shows a deepened periodic pattern with increased amplitude and decreased frequency of bursts (every 6-12 seconds). Further worsening of the picture leads to a very-low-voltage or isoelectric EEG.

As used herein, a "derivative" of a HIP/PAP protein encompasses polypeptides comprising an amino acid sequence having at least 90% amino acid identity with a polypeptide selected from the group consisting of SEQ ID N° 1 to 3..

Comprises/comprising and grammatical variations thereof when used in this specification are to be taken to specify the presence of the stated features, integers, steps or components or groups thereof, but do not preclude the presence or addition of one or more other features, integers, steps, components or groups thereof.

A HIP/PAP protein derivative encompasses proteins comprising an amino acid sequence of at least 90 %, and preferably of at least 91%, 92%, 93%, 94%, 95%, 96%, 97%, 98%, 99%, or more amino acid identity with a polypeptide selected from the group consisting of the polypeptides of SEQ ID N° 1 to 3.

In some embodiments, a HIP/PAP protein derivative encompasses a protein having a strong amino acid identity with a HIP/PAP protein selected from the group consisting of SEQ ID N° 1-3, and possessing, as compared with the HIP/PAP protein of reference, one or more additions, substitutions or deletions of an amino acid residue. According to these embodiments, the said HIP/PAP protein derivative may comprise 1, 2, 3, 4, 5, 6, 7, 8, 9, 10, 11, 12, 13, 14, 15, 16, 17, 18, 19, 20 or more additions, substitutions or deletions of an amino acid residue, as compared with the amino acid sequence of the HIP/PAP protein of reference. According to these embodiments, the said HIP/PAP protein may possess not more than 25 additions, substitutions or deletions of an amino acid residue, as compared with the amino acid sequence of the HIP/PAP protein of reference.

As intended herein, a HIP/PAP protein derivative is biologically active. As intended herein, a HIP/PAP protein derivative encompasses a biologically active portion of a HIP/PAP protein.

A "biologically active" derivative of a HIP/PAP protein includes a HIP/PAP-derived peptide that possesses one or more of the following biological activities :
- (i) preventing *in vitro* the N-methyl-D-aspartate (NMDA)-induced neuronal cell death in primary mice neuronal cultures;
- (ii) preventing *in vivo* the excitotoxic lesions caused by the glutamanergic analogue ibotenate in the developing brain, e.g. the mouse developing brain;
- (iii) possessing an *in vivo* rescuing effect on the excitotoxic lesions caused by the glutamanergic analogue ibotenate in the developing brain, e.g. the mouse developing brain;
- (iv) effecting neuroprotection against neonatal neuronal cell death induced by a reactive oxygen species like hydrogen peroxide (H₂O₂).; and
- (v) inducing changes in the production, phosphorylation and distribution of Gap-43

A "biologically active" derivative of a HIP/PAP protein with a biological activity selected among (i)-(v) above, is not necessarily endowed with the same order of magnitude of the said biological activity(ies), provided that the said HIP/PAP protein derivative possesses one or more of the biological activities (i)-(iv) above at a detectable level, using the assays disclosed in the examples herein.

In some embodiments, a HIP/PAP protein or a derivative thereof, including a biologically active portion of HIP/PAP as described above, can be isolated from cell or tissue sources by an appropriate purification scheme using standard protein purification techniques.

In other embodiments, a HIP/PAP protein or a derivative thereof, including a biologically active portion of HIP/PAP as described above, may be produced by recombinant DNA techniques that are familiar to one skilled in the art.

According to further embodiments, a HIP/PAP protein or a derivative thereof, including a biologically active portion of HIP/PAP as described above, may be synthesised chemically using standard peptide synthesis techniques.

An isolated or purified HIP/PAP protein or derivative thereof, including a biologically active portion of HIP/PAP as described above, is substantially free of cellular material or other contamination proteins from the cell or tissue source from which the said protein is derived, or substantially free from chemical precursors when chemically synthesised.

HIP/PAP protein derivatives also encompass HIP/PAP chimeric or fusion proteins. As used herein, a chimeric protein or a fusion protein comprises the polypeptides cited above which are fused to a non-HIP/PAP polypeptide. Within the fusion protein, the HIP/PAP polypeptide and the non-HIP/PAP polypeptide are fused to each other. The non-HIP/PAP polypeptide can be fused to the N-terminus or to the C-terminus of the HIP/PAP polypeptide.

For example, in one embodiment, the fusion protein is a GST-HIP/PAP fusion protein in which the HIP/PAP sequence is fused to the C-terminus of the GST sequence. Such fusion proteins can facilitate the purification of recombinant HIP/PAP.

In all cases the fusion proteins of the invention are "biologically active" according to the previous definition of this expression herein, which includes that the said fusion proteins possess the same biological activity as the HIP/PAP of SEO ID N°3.

To determine the percent identity of two amino acid sequences for the purposes of the instant invention, the sequences are aligned for optimal comparison purposes. For example, gaps can be introduced in one or both of a first and a second amino acid sequence for optimal alignment and non-homologous sequences can be disregarded for comparison purposes.

For optimal comparison purposes, the percent identity of two amino acid sequences can be achieved with CLUSTAL W (version 1.82) with the following parameters: (1) CPU MODE = ClustalW mp; (2) ALIGNMENT = « full » ; (3) OUTPUT FORMAT = « aln w/numbers » ; (4) OUTPUT ORDER = « aligned » ; (5) COLOR ALIGNMENT = « no » ; (6) KTUP (word size) = « default » ; (7) WINDOW LENGTH = « default » ; (8) SCORE TYPE = « percent » ; (9) TOPDIAG = « default » ; (10) PAIRGAP = « default » ; (11) PHYLOGENETIC TREE/TREE TYPE = « none » ; (12) MATRIX = « default » ; (13) GAP OPEN = « default » ; (14) END GAPS = « default » ; (15) GAP EXTENSION = « default » ; (16) GAP DISTANCES = « default » ; (17) TREE TYPE = « cladogram » et (18) TREE GRAP DISTANCES = « hide ».

Biologically active portions of HIP/PAP include peptides comprising amino acid sequences sufficiently homologous to the full length amino acid sequence of HIP/PAP of any one of SEQ ID N°1 to 3, which include the same number of amino acids than the corresponding full length HIP/PAP, and exhibit at least the same biological activity than HIP/PAP.

Biologically active portions of HIP/PAP include further peptides comprising amino acid sequences sufficiently homologous to the full length amino acid sequence of HIP/PAP of any one of SEQ ID N°1 to 3, which include more amino acids than the corresponding full length HIP/PAP, and exhibit at least the same biological activity than HIP/PAP.

In addition to naturally occurring allelic variants of the biologically active portion of HIP/PAP sequences that exist in mammals, the person skilled in the art will further appreciate that changes can be introduced by mutation into the nucleotide sequence of any one of SEQ ID N°1 to 3, thereby leading to changes in the amino acid sequence of HIP/PAP without altering the biological activity of HIP/PAP.

In addition, substitutions of non-essential amino acid can be made in the sequences corresponding to a HIP/PAP protein. A "non essential" amino acid residue is an amino acid residue that can be altered from the wild type sequence of HIP/PAP without altering the biological activity, whereas an "essential" amino acid residue is required for biological activity.

Generally, when used for preventing or treating a brain injury, or for any other HIP/PAP uses disclosed herein, a HIP/PAP protein or a protein derivative thereof is comprised in a pharmaceutical composition at an effective amount, in combination with one or more physiologically acceptable excipients.

Without wishing to be bound to any particular theory, the inventors believe that the complete HIP/PAP protein of sequence SEQ ID N°2, i.e. a polypeptide comprising the CRD sequence, and a pro-peptide, leads to a best folding of said protein, particularly when said protein is produced through DNA recombinant techniques in eukaryotic cells that have been transfected with an expression cassette encoding it. Incidentally, a correct folding of the therapeutically active HIP/PAP may improve biological efficiency for the pharmaceutical composition comprising said protein, for preventing or treating a brain injury caused by hypoxia, as compared to a composition comprising only a portion of the protein.

However, other forms of a HIP/PAP protein including the polypeptide forms of SEQ ID N°1 and 2, as well as any one of the HIP/ PAP derivatives described in the present specification, may be used *in vitro* and *in vivo,* even if these polypeptides do not necessarily consist of the most preferred embodiments of the instant invention.

As previously specified in the present description, the instant invention relates notably to the use of a HIP/PAP protein or a protein derivative thereof for preventing or treating an adult brain injury caused by a brain hypoxia. This invention thus pertains to the use of a HIP/PAP protein or a protein derivative thereof for preventing or treating an adult brain injury caused by a brain hypoxia.

Without wishing to be bound by any particular theory, the inventors believe that the various effects of the HIP/PAP protein on the *in vitro* and *in vivo* neonatal brain injury that are disclosed herein may also be useful in very specific patho-physiological situations of the adult brain selected from the group consisting of cerebrovascular accidents and traumatic brain injury.

Thus, in certain embodiments according to the invention, the said adult brain injury consists of a cerebrovascular accident, which may be also termed stroke.

A cerebrovascular accident or stroke consists of a rapidly loss of brain functions due to a disturbance in the blood vessels supplying blood to the brain. A cerebrovascular accident encompasses (i) an ischemia caused by thrombosis or embolism, which may be also termed "ischemic stroke" and (ii) an ischemia due to a haemorrhage, which may be also termed "hemorrhagic stroke". Like neonatal brain injury, a cerebrovascular accident or stroke may cause permanent neurological damage and may lead to death. According to the World Health Organisation, a cerebroventricular accident or stroke consists of a neurological deficit of cerebrovascular cause that persists beyond 24 hours or is interrupted by death within 24 hours.

A cerebrovascular accident or stroke encompasses thrombotic stroke, embolic stroke, ischemia caused by a cardiac dysfunction, systemic hypoperfusion, venous thrombosis and intracerebral haemorrhage.

In certain other embodiments according to the invention, the said adult brain injury consists of a traumatic brain injury, also termed intracranial injury.

Traumatic brain injury (TBI) occurs when physical trauma injures the brain and is generally classified as mild, moderate, or severe, depending on the extent of loss of consciousness, loss of memory, and score on a neurological scale following the injury. Diagnosis and scaling of traumatic brain injury is conventionally performed by a method selected from the group consisting of Glascow Coma Scale (GCS) (Arlinghaus et al., 2005, Textbook of Traumatic Brain Injury, Washington, DC : American Psychiatric Association, pp; 59-62), a method of determination of the duration of post-traumatic amnesia (PTA), and a method of scaling the loss of consciousness (LOC) (Rao et al., 2000, Psychosomatics, Vol. 41 (n°2) : 95-103). Among the physiological similarities between Trauma brain injury and neonatal brain injury, TBI is accompanied by haemorrhages due to rupture of blood vessels which causes hypoxia.

According to the present invention, HIP/PAP protein or a protein derivative thereof may be used for preventing or treating traumatic brain injuries having GCS classification scores ranging form 3 to 15. Thus, HIP/PAP protein or a protein derivative thereof may be used for preventing or treating traumatic brain injuries selected from the group consisting of "mild" TBI (GSC score ranging from 13 to 15), moderate TBI (GCS score ranging from 9 to 12) and severe TBI (GSC score ranging from 3 to 8). Typically, mild TBI have a GCS score ranging from 13 to 15, a PTA score less than one hour and a LOC score less than 30 minutes. Typically, moderate TBI have a GCS score ranging from 9 to 12, a PTA score ranging from 30 minutes to 24 hours and a LOC score ranging from 1 to 24 hours. Typically, severe TBI have a GCS score ranging from 3 to 8, a PTA score of more than one day and a LOC score of more than one day.

As already mentioned in the present specification, because of the HIP/PAP activity on the Gap-43 production or phosphorylation that has been shown herein, a HIP/PAP protein or a derivative thereof may also be used *in vitro* and *in vivo* for modulating, enhancing or improving neuronal growth or plasticity mediated by Gap-43, a neuronal growth-related protein.

This invention thus also concerns the use of the HIP/PAP protein or a protein derivative thereof for preventing or treating a disorder or a disease involving a defect or a deregulation of Gap-43 production or a defect or a deregulation of Gap-43 phosphorylation, which includes Alzheimer's disease.

It is worth mentioning that HIP/PAP-related proteins have already been alleged in the art to be useful for acting on Alzheimer's disease, but without any starting experimental result that would technically support such activity allegations.

In contrast, the biological activity of a HIP/PAP protein on the production or on the phosphorylation of the Gap-43 protein supports for the fist time the usefulness of the said HIP/PAP protein for preventing or treating Alzheimer's disease.

Indeed, it is well known from the one skilled in the art that a defect in the production or in the phosphorylation of the Gap-43 protein occurs in Alzheimer's disease. Notably, it has been shown in the art that the production of an inactive Gap-43 protein is involved in patients affected with an Alzheimer's disease. Also, an altered phosphorylation of Gap-43 has previously been shown in the art in patients affected with an Alzheimer's disease.

Thus, this invention also relates to the use of a HIP/PAP protein or a derivative thereof for preventing or treating an Alzheimer's disease.

The present invention also concerns a method for preventing or treating an Alzheimer's disease comprising a step of administering, to a patient in need thereof, a HIP/PAP protein or a derivative thereof.

Because it has been shown in the examples herein that a HIP/PAP protein has the ability to rescue neuronal cells *in vitro,* then a HIP/PAP protein or a derivative thereof has also been proved to be useful as an agent for maintaining or improving the viability or the growth of cultured neuronal cells. Then, the results of the examples herein support the usefulness of a HIP/PAP protein or of a derivative thereof as a neuronal cell culture agent that may be added in a neuronal cell culture medium.

This invention also encompasses the use of a HIP/PAP protein for the *in vitro* culturing of neuronal cells, including for primary cultures of neuronal cells and for cultures of neuronal cell lines.

The present invention also pertains to a culture medium for neuronal cells comprising a HIP/PAP protein or a derivative thereof. Apart the presence of a HIP/PAP protein or of a derivative thereof, the qualitative and quantitative constitution of an appropriate culture medium is easily determined by the one skilled in the art. Illustratively, the HIP/PAP protein or the derivative thereof may simply be added to a known culture medium suitable for neuronal cells, e.g. in a RPMI 199 culture medium.

The said culture medium comprises an effective amount of the said HIP/PAP protein or of the said derivative thereof. The final concentration of a HIP/PAP protein or a derivative thereof in a culture medium above preferably ranges from 1 ng/mL to 10 mg/ml.

According to the instant invention, regarding the *in* vivo uses, the HIP/PAP protein or a protein derivative thereof is preferably comprised as an active ingredient in a pharmaceutical composition.

The said pharmaceutical composition comprises the HIP/PAP protein, or a protein derivative thereof, and one or more pharmaceutically acceptable excipients.

The said pharmaceutical composition comprises en effective amount of the HIP/PAP protein, or a protein derivative thereof.

Usually, a pharmaceutical composition that may be used according to the invention comprises from 0.1 % by weight to 99.9% by weight of the HIP/PAP protein, or a protein derivative thereof, based on the total weight of the pharmaceutical composition. Thus, a pharmaceutical composition according to the invention may comprise at least 0.5%, 1%, 2%, 3%, 4%, 5%, 6%, 7%, 8%, 9%, 10%, 20%, 30%, 40%, 50%, 60%, 70%, 80%, 90% by weight or more of the HIP/PAP protein, or a protein derivative thereof, based on the total weight of the pharmaceutical composition. Also, a pharmaceutical composition according to the invention may comprise up to 10% 20%, 30%, 40%, 50%, 60%, 70%, 80%, 90%, 91%, 92%, 93%, 94%, 95%, 96%, 97%, 98%, 99%, 99.5% by weight or less of one or more pharmaceutically acceptable excipients, based on the total weight of the pharmaceutical composition.

The therapeutically effective dose of the HIP/PAP protein or a protein derivative thereof will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. The clinician will administer the HIP/PAP protein or a protein derivative thereof until a dosage is reached that achieves the desired effect for treatment of the condition in question.

As used herein more particularly, an effective amount of the HPI/PAP protein or of the protein derivative thereof consists of the amount of HIP/PAP protein or derivative thereof that can at least partially reverse the physical damages caused to the brain during a neonatal brain injury. Particularly, an effective amount of the HPI/PAP protein or of the protein derivative thereof consists of the amount of HIP/PAP protein or derivative thereof that reduces or blocks neuronal cell death occurring during neonatal brain injury.

Starting form the results shown in the examples herein, the effective amount of the HIP/PAP protein or of the protein derivative thereof may range from about 0.1 µg/kg of body weight and to about 100 mg/ kg of body weight. Thus, according to the invention, an effective amount of the HIP/PAP protein or of the protein derivative thereof encompasses amounts of at least 1 µg/kg, 2 µg/kg, 3 µg/kg, 4 µg/kg, 5 µg/kg, 6 µ/kg, 7 µg/kg, 8 µg/kg, 9 µg/kg, 10 µg/kg, 15 µg/kg, 20 µ/kg, 20, µg/kg, 30 µg/kg, 40 µg/kg, 50 µg/kg, 60 µg/kg, 70 µg/kg, 80 µg/kg, 90 µg/kg, 100 µg/kg, 200 µg/kg, 300 µg/kg, 400 µg/kg, 500 µg/kg, 600 µg/kg, 700 µg/kg, 800 µ/kg, 900 µg/kg, 1 mg/kg or more of body weight of the patient.

For the purpose of the instant invention the "patient" encompasses the neonate patient as well as the pregnant patient.

To prevent or treat a neonatal brain injury to the foetus body, a pharmaceutical composition according to the invention is preferably administered to the pregnant adult body. However, in some embodiments, the said pharmaceutical composition may be administered directly to the foetus body, using specific endoscopy medical instrumentation.

To prevent or treat a neonatal brain injury of the neonate during delivery or subsequent to delivery, as well as to prevent or treat a brain injury of the adult caused by hypoxia, a pharmaceutical composition according to the invention is administered to the neonate body or to the adult body, respectively.

In certain embodiments, a pharmaceutical composition according to the invention is administered systemically.

In certain other embodiments, a pharmaceutical composition according to the invention is administered locally via an intracranial route, especially to the foetus body or the neonate body where skull bone formation is not terminated.

The route of the HIP/PAP protein or of the protein derivative thereof administration is in accord with known methods, e.g., by injection or infusion by intravenous, intramuscular, intracerebral, intraperitoneal, intracerobrospinal, subcutaneous, oral, topical, or inhalation routes, or by sustained-release systems as noted below.

In some embodiments, the HIP/PAP protein or of the protein derivative thereof, or alternatively the pharmaceutical composition containing the same, is administered as a single dose.

In some other embodiments, the HIP/PAP protein or of the protein derivative thereof, or alternatively the pharmaceutical composition containing the same, is administered as a plurality of doses at determined time intervals, e.g. starting from the time of brain injury diagnosis or from the time at which brain injury expectation is diagnosed, and until the time upon which the treated body is out of danger.

In certain embodiments of a pharmaceutical composition according to the invention, the HIP/PAP protein or of the protein derivative thereof, may be combined with one or more other active ingredients useful for preventing or treating a brain injury caused by a brain hypoxia, including one or more active ingredients already known in the art for being useful for preventing or treating a neonatal brain injury.

Therapeutic compositions according to the invention may be prepared for storage by mixing the desired molecule having the appropriate degree of purity with optional pharmaceutically acceptable carriers, excipients, or stabilizers (Remington's Pharmaceutical Sciences, 16th edition, Osol, A. ed. (1980)), in the form of lyophilized formulations or aqueous solutions. Acceptable carriers, excipients, or stabilizers are non toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatine, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN.TM., PLURONICS.TM. or polyethylene glycol (PEG).

Additional examples of such carriers include ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms of the HIP/PAP or derivative thereof substances according to the invention include polysaccharides such as sodium carboxymethylcellulose or methylcellulose, polyvinylpyrrolidone, polyacrylates, polyoxyethylene-polyoxypropylene-blo- ck polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The HIP/PAP protein or the protein derivative thereof will typically be formulated in such vehicles at a concentration of about 0.1 mg/ml to 100 mg/ml.

The HPI/PAP protein or the protein derivative thereof to be used for *in vivo* administration must be sterile. This is readily accomplished by filtration through sterile filtration membranes, prior to or following lyophilization and reconstitution. The HIP/PAP protein or of the protein derivative thereof will ordinarily be stored in lyophilized form or in solution if administered systemically. If in lyophilized form, the HPI/PAP protein or the protein derivative thereof is typically formulated in combination with other ingredients for reconstitution with an appropriate diluent at the moment of use. An example of a liquid formulation of the HIP/PAP protein or the protein derivative thereof is a sterile, clear, colorless unpreserved solution filled in a single-dose vial for subcutaneous injection. Preserved pharmaceutical compositions suitable for repeated use may contain, for example, depending mainly on the indication and type of polypeptide:
a) the HIP/PAP protein or the protein derivative thereof;
b) a buffer capable of maintaining the pH in a range of maximum stability of the polypeptide or other molecule in solution, preferably about 4-8;
c) a detergent/surfactant primarily to stabilize the polypeptide or molecule against agitation-induced aggregation;
d) an isotonifier;
e) a preservative selected from the group of phenol, benzyl alcohol and a benzethonium halide, e.g., chloride; and
f) water.

If the detergent employed is non-ionic, it may, for example, be polysorbates (e.g., Polysorbate®, Tween®.) 20,80, etc.) or poloxamers (e.g., Poloxamer®. 188). The use of non-ionic surfactants permits the formulation to be exposed to shear surface stresses without causing denaturation of the polypeptide. Furthermore, such surfactant-containing formulations may be employed in aerosol devices such as those used in a pulmonary dosing, and needleless jet injector guns (see, e.g., EP 257,956).

An isotonifier may be present to ensure isotonicity of a liquid composition of the HIP/PAP protein or the protein derivative thereof, and includes polyhydric sugar alcohols, preferably trihydric or higher sugar alcohols, such as glycerin, erythritol, arabitol, xylitol, sorbitol, and mannitol. These sugar alcohols can be used alone or in combination. Alternatively, sodium chloride or other appropriate inorganic salts may be used to render the solutions isotonic.

The buffer may, for example, be an acetate, citrate, succinate, or phosphate buffer depending on the pH desired. The pH of one type of liquid formulation of this invention is buffered in the range of about 4 to 8, preferably about physiological pH.

The preservatives phenol, benzyl alcohol and benzethonium halides, e.g., chloride, are known antimicrobial agents that may be employed.

Compositions of the therapeutic HIP/PAP protein or protein derivative thereof are generally placed into a container having a sterile access port, for example, an intravenous solution bag or vial having a stopper pierceable by a hypodermic injection needle. The formulations are preferably administered as repeated intravenous (i.v.), subcutaneous (s.c.), or intramuscular (i.m.) injections, or as aerosol formulations suitable for intranasal or intrapulmonary delivery (for intrapulmonary delivery see, e.g., EP 257,956).

The HIP/PAP protein or the protein derivative thereof can also be administered in the form of sustained-released preparations. Suitable examples of sustained-release preparations include semipermeable matrices of solid hydrophobic polymers containing the protein, which matrices are in the form of shaped articles, e.g., films, or microcapsules. Examples of sustained-release matrices include polyesters, hydrogels (e.g., poly(2-hydroxyethyl-methacr- ylate) as described by Langer et al., J. Biomed. Mater. Res., 15: 167-277 (1981) and Langer, Chem. Tech., 12: 98-105 (1982) orpoly(vinylalcohol)), polylactides (U.S. Pat. No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer et al., supra), degradable lactic acid-glycolic acid copolymers such as the Lupron Depot.TM. (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C., resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release HIP/PAP protein or protein derivative thereof also include liposomally entrapped polypeptides.. Liposomes containing the polypeptide of interest are prepared by methods known per se: DE 3,218,121; Epstein et al., Proc. Natl. Acad. Sci. USA. 82: 3688-3692 (1985); Hwang et al., Proc. Natl. Acad. Sci. USA, 77: 4030-4034 (1980); EP 52,322; EP 36,676; EP 88,046; EP 143,949; EP 142,641; Japanese patent application 83-118008; U.S. Pat. Nos. 4,485,045 and 4,544,545; and EP 102,324. Ordinarily the liposomes are of the small (about 200-800 Angstroms) unilamellar type in which the lipid content is greater than about 30 mol. % cholesterol, the selected proportion being adjusted for the optimal therapy.

The therapeutically effective dose of a HIP/PAP protein or protein derivative thereof will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. The clinician will administer the HIP/PAP protein or a protein derivative thereof until a dosage that achieves the desired effect for treatment of the condition in question is reached.

The route of administration is in accord with known methods, e.g. administration by injection or infusion by intravenous, intramuscular, intracerebral, intraperitoneal, intracerobrospinal, subcutaneous, intraocular, intraarticular, intrasynovial, intrathecal, oral, topical, or inhalation routes, or administration by sustained-release systems as noted below. The HIP/PAP or a protein derivative thereof also are suitably administered by intralesional or perilesional routes, to exert local as well as systemic therapeutic effects. Examples of pharmacologically acceptable salts of molecules that form salts and are useful hereunder include alkali metal salts (e.g., sodium salt, potassium salt), alkaline earth metal salts (e.g., calcium salt, magnesium salt), ammonium salts, organic base salts (e.g., pyridine salt, triethylamine salt), inorganic acid salts (e.g., hydrochloride, sulfate, nitrate), and salts of organic acid (e.g., acetate, oxalate, p-toluenesulfonate).

The HPI/PAP protein or the protein derivative thereof may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, supra.

Sustained-release preparations may be prepared. Suitable examples of sustained-release preparations include semipermeable matrices of solid hydrophobic polymers containing the antibody, which matrices are in the form of shaped articles, e.g. films or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C., resulting in a loss of biological activity and possible changes in immunogenicity. Various rational strategies of stabilization can be devised, depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

The instant invention is further illustrated by, without being limited by, the examples below.

### EXAMPLES

### Example 1 : In vitro effects of a HIP/PAP protein in a model of neonatal brain injury

### A. Materials and Methods

### Cortical Neurons Culture.

Primary cortical neurons cultures were prepared from E14.5 embryonic mice. Briefly, foetuses were removed from the uterus and placed in a sterile Petri dish. Brains were cut and placed in HBSS 1x with 1 M HEPES (Invitrogen). Using a dissecting microscope, brains, meninges, basal ganglia and hippocampus were dissected out and neocortex were put in Neurobasal medium containing B27 supplement (Invitrogen). Then, neocortex were dissociated with 0.25% Trypsin - 0.02% EDTA and incubated with DNase 1 (Sigma St-Quentin Fallavier, France). Cells were platting with MEM1X and 10% Horse serum at a density of 80000 cells/well in 96 wells plates coated with poly-DL-ornithine (Sigma St-Quentin Fallavier, France). Two to four hours after platting, the medium was removed and substituted with Neurobasal plus B27. Three days later, cells were treated with 5 µM of AraC (cytosine arabinoside) to curb glial proliferation. Between DIV10 and DIV12, neurons were treated for 8 hours with either one or the other product:
(i): PBS;
(ii): 3µg/ml or 5 µg/ml of ALF-5755;
(iii): 300 µM of NMDA;
(iv): 300 µM of NMDA plus 10 µM of MK801 or
(v): 300 µM of NMDA and 3µg/ml or 5 µg/ml of ALF-5755.

### Measurement of Cell viability

Cell viability was monitored using the MTS/Formazan assay (CellTiter 96 Aqueous One Solution Cell Proliferation Assay; Promega, Charbonnieres, France).

### Western blot analysis.

Total proteins were extracted from treated neuronal cultures (see below) and from the right neopallium of pups which has received at P5 intraparenchymal injections of either PBS, 10 µg of ibotenate, 0.3 µg of ALF-5755 (which has been determined as the best effective concentration of ALF-5755) or 10 µg ibotenate with 0.3 µg of ALF-5755. Pups were killed 4, 24, 48 or 120 hours after drugs administration. Proteins were extracted according to the protocol of a commercially available kit (Cell Signaling Technology).

Anti-Total-Gap43 and anti-Ser41-Phospho-Gap43 were used at a concentration of 1/1000. To standardize the protein expression among samples, we used an anti-b-tubulin goat antibody (Santa Cruz Biotechnology) at a concentration of 1/10000. Western blot experiments were run in duplicate. Differences in protein concentrations between samples were estimated using ImageJ.

### Immunocytochemistry.

For Total Gap-43, PhosphoGap43 and growth cone (2G13) detections, treated cell cultures were fixed in 4% PFA and reacted with chicken anti-Total-Gap43 antibody (Interchim; 1/200), rabbit anti-Ser41-PhosphoGap43 antibody (Novus Biologicals 1/200), mouse anti-Growth Cone 2G13 antibody (Novus Biologicals 1/200). Detection of labelled antigens was performed with a Cy3-conjugated donkey anti-chicken antibody, AMCA-conjugated donkey anti-rabbit antibody and Alexa 488-conjugated donkey anti-mouse antibody (Jackson Immunoresearch).

### Statistical Analysis

To study the effect of each treatment, the data were analyzed with a Kruskal-Wallis test.

### B. Results

A recombinant human HIP/PAP protein was produced in an *E. coli* bacterial system, and then has been purified. The resulting product was called ALF-5755. Compared with HIP/PAP SEQ ID N°2, ALF-5755 of SEQ ID N°3has one single extra amino acid, a methionine, in the NH2 terminal end.

Biochemical, biological, and functional studies of ALF-5755 have been performed. They established that ALF-5755 exhibited the same biological activities, especially, mitogenic and antiapoptotic activities, as those previously demonstrated to pertain to HIP/PAP in several different animal models.

The neuroprotective efficacy of ALF-5755, *in vitro* against N-methyl-D-aspartate (NMDA)-induced cell death has been studied in primary mice cortical neuronal cultures.

The ALF-5755 *in vitro* neuroprotection properties have been evaluated, using a well-established model of primary neuronal cultures (neurons from cerebral hemisphere of E14 Swiss mice exposed to NMDA). The experiments were performed between day 8 and day 12 from the onset of the culture. Doses of HIP/PAP (ranging from 150 ng/ml to 10 µg/ml) were added to the culture medium immediately after or 3 hours after the beginning of an 8-hour intoxication by 300 µM of NMDA or by 10, 30, 100 µM of H2O2. Cell viability was then measured using a MTT assay.

The neuronal death after exposure to NMDA alone (without treatment with ALF-5755) was of 60% of the neurons. Treatment with ALF-5755 caused a significant, dose-dependent decrease of the proportion of neuronal death. The decrease in neuronal death was maximum (35% of neuronal death against 45% without ALF-5755) at 5 µg/ml of ALF-5755 when added immediately after the beginning of the insult. A neuroprotective effect of ALF-5755 was also observed when it was added 3 hours after the beginning of the insult.

Exposure to H₂O₂ alone induces a dose-dependent diminution of cell survival, which is maximal at 100µM exposure (64% of neuronal death). Treatment with ALF-5755 totally or partially reverses the toxicity of H₂O₂. This protective effect is maximal for 3µg/ml of ALF-5755. Protective effects of ALF-5755 were comparable to those observed with catalase. Protective effects of ALF-5755 are observed whether the drug is added immediately after H₂O₂ or 3 hours after the insult.

Immunocytochemistry (ICC) studies and western blot (WB) analyses were performed on neuronal cultures to determine the effects of ALF-5755 on the production, phosphorylation status at serine 41 (S41) and distribution of GAP-43, a protein involved in axonal growth and neuronal plasticity.

When added immediately after NMDA, ICC demonstrates that ALF-5755 induces a fascicular distribution of GAP-43-labelled neuronal processes. This effect is optimal at 5µg/ml and is not observed in control cultures, in cultures treated with NMDA or H₂O₂, or in cultures treated with H₂O₂ and ALF-5755. Furthermore, ICC shows that ALF-5755 induces an increased expression of phospho-GAP-43 in neuronal processes, when compared to control cultures or to cultures treated with NMDA or H202 alone.

Western blot analyses of extracts from cultured neurons allowed to quantify changes of total GAP-43 and phospho-GAP-43 levels. NMDA did not induce any significant changes in the amount of GAP-43. In contrast, ALF-5755 alone induced a dose-dependent decrease of total GAP-43 but a significant increase of the proportion of phosphorylated GAP-43 (phospho GAP-43 / total Gap-43). The effect of ALF-5755 on GAP-43 was still more pronounced in cultures exposed to NMDA. These changes of in the phosphorylation status of GAP-43 induced by ALF-5755 could explain the fasciculation observed in cultures exposed to ALF-5755.

Altogether these data support the hypothesis that ALF-5755 prevents excitotoxic and oxidative neuronal cell death in vitro. Morever, ALF-5755 induces plasticity-like changes including GAP-43 phosphorylation and fasciculation.

### Example 2 : In vivo effects of a HIP/PAP protein in a model of neonatal brain injury

### A. Materials and Methods

### Animals

Swiss mice of both sexes were used in this study. Experimental protocols were approved by the institutional review committee and meet the guidelines of the 'Institut National de la Santé et de la Recherche Médicale'.

### Drugs

Ibotenate (Sigma, St-Quentin Fallavier, France), ALF-5755, NMDA (Tocris, Bristol, UK) and MK801 (Sigma, St-Quentin Fallavier, France) were diluted in phosphate-buffered saline (PBS).

### Administration of Excitotoxic Drugs

Excitotoxic brain lesions were induced by injecting ibotenate into developing mouse brains, as previously described (Marret et al., 1995; Gressens et al., 1997, 1998; Bac et al., 1998; Dommergues et al., 2000; Tahraoui et al., 2001; Laudenbach et al., 2001, 2002; Husson et al., 2002). Briefly, mouse pups were injected intracerebrally (into the neopallial parenchyma) on day P5. Intraparenchymal injections were performed with a 25-gauge needle on a 50 µl Hamilton syringe mounted on a calibrated microdispenser. The needle was inserted 2 mm under the external surface of scalp skin in the frontoparietal area of the right hemisphere, 2 mm from the midline in the lateral-medial plane, and 3 mm anterior to bregma in the rostro-caudal plane. Two 1 µl doses of ibotenate, or ibotenate plus ALF-5755 or PBS were injected at 20 s intervals. The first bolus corresponds to an injection localized in the white matter and the second bolus represents the cortical injection. 10 µg ibotenate or 10 µg ibotenate plus ALF-5755 (range: 0.0015 µg-0.15 µg) or PBS was administered to each pup.

For some pups, ALF-5755 has been injected intraperitonealy immediately or 3 hours after ibotenate injection at different concentrations (from 0.0015 µg to 1.5 µg).

### Experimental Groups

Pups from at least two different litters were used in each experimental group, and data were obtained from two or more successive experiments.

Three different groups were constituted depending on how (intraparachymal co-administered with ibotenate, intraperitoneal injected immediately or 1 to 5 hours after ibotenate injection) ALF-5755 was injected.

The first P5 pups group received an intraparenchymal injection of ibotenate with PBS (vehicle control) or with 0.003, 0.015, 0.03 or 0.3 µg of ALF-5755.

In the second set of experiments designed to compare the intraparenchymal injection with the intraperitoneal injection of ALF-5755, P5 pups received an intraparachymal injection of ibotenate with PBS and then an intraperitoneal injection of ALF-5755 at several concentrations (0.0015, 0.0075, 0.015, 0.075, 0.15, 0.75 or 1.5 µg of ALF-5755).

To know if ALF-5755 can repair, instead of preventing, ibotenate lesions, the third P5 mouse pups group received a delayed intraperitoneal injection of 0.75 or 1.5 µg of ALF-5755 3 hours after intraparenchymal injection of ibotenate. One or five hours after ibotenate injection, a single ALF-5755 concentration was tested intraperitonealy (1.5 µg).

### Determination of Lesion Size

12 mouse pups were used in each experimental group. Mouse pups were sacrificed by decapitation 120 h following the excitotoxic challenge. Brains were immediately fixed in 4% formalin for at least 4 days. Following embedding in paraffin, we cut 20 µm-thick coronal sections. Every third section was stained with cresyl-violet. Previous studies (Marret et al., 1995; Gressens et al., 1997; Husson et al., 2002) have shown an excellent correlation between the maximal size of the lesion in the lateral-medial and fronto-occipital axes of the excitotoxic lesions. This permitted an accurate and reproducible determination of the maximal fronto-occipital diameters of the lesions, which equals the number of sections where the lesion was present multiplied by 20 µm.

### B. Results

The neuroprotective potential of ALF-5755 *in vivo* was evaluated by means of a mouse model of neonatal excitotoxic brain lesions mimicking brain damage associated with cerebral palsy.

Brain damage was induced by ibotenate, a glutamanergic agonist acting on NMDA and metabotropic glutamate receptors. When injected into the murine neopallium at postnatal day (P) 5, ibotenate produced dramatic neuronal loss in neocortical layers and large periventricular white matter cysts resembling those seen in periventricular leukomalacia, which is a lesion appearing in numerous human premature newborns.

ALF-5755 was either co-injected with ibotenate intracerebrally at P5, or injected ALF-5755 intraperitoneally immediately, or 3 hours after ibotenate. The analysis of brain lesions was performed at P10.

Co-injection of ALF-5755 with ibotenate reduced white-matter and cortical gray-matter lesions by 37% and 67%, respectively, for a dose of 150 ng ALF-5755 (as compared with pups treated with ibotenate alone). A good (50% decrease of the size of the cerebral lesions) neuroprotection was also observed when the injection of ALF-5755 was performed intraperitoneally immediately, or 3 hours after insult. This establishes the efficacy of a single injection of ALF-5755, even delayed, via the systemic route. This also indicates that ALF5755 administered either intra-cerebrally or at the periphery can exhibit a therapeutic effect in neurological diseases and related diseases as defined above.

Further confirming the *in vitro* data, western blot analysis showed that ALF-5755, alone or in combination with ibotenate, induces a significant decrease in total GAP-43, but a significant and very important increase in the proportion of phosphorylated GAP-43 when compared to controls or animals injected with ibotenate alone.

## Claims

**1.** The use of the HIP/PAP protein or a protein derivative thereof for preventing or treating a disease selected from the group consisting of a neonatal brain injury, an adult brain injury caused by a brain hypoxia and a disease involving a defect in the production or in the phosphorylation of Gap-43.

**2.** The use according to claim 1, wherein the said brain injury consists of a neonatal brain injury.

**3.** The use according to claim 2, wherein neonatal brain injury encompasses a pathological state selected from the group consisting of foetal hypoxemia, perinatal hypoxemia, foetal ischemia, perinatal ischemia, and an excess release of excitatory neurotransmitters.

**4.** The use according to claim 1, wherein the said brain injury consists of a cerebrovascular accident.

**5.** The use according to claim 1, wherein the said brain injury consists of a traumatic brain injury.

**6.** The use according to claim 1, wherein the said disease involving a defect in the production or in the phosphorylation of Gap-43 consists of Alzheimer's disease.

**7.** The use according to any one of claims 1 to 6, wherein the HIP/PAP protein or a protein derivative thereof modulates the structural remodelling or plasticity of central or peripheral neuronal cell.

**8.** The use according to any one of claims 1 to 6, wherein the HIP/PAP protein or derivative thereof comprises an amino acid sequence having at least 90% amino acid identity with a polypeptide selected from the group consisting of the polypeptides of SEQ ID N°1 to 3.

**10.** The use according to any one of claims 1 to 6, wherein the said HIP/PAP protein derivative is selected from the group consisting of a biologically active portion of a HIP/PAP protein and HIP/PAP chimeric or fusion proteins.

**11.** The use of a HIP/PAP protein for the *in vitro* culturing of neuronal cells.

**12.** A culture medium for neuronal cells comprising a HIP/PAP protein or a derivative thereof.


SEQUENCE LISTING

<110>  ALFACT INNOVATION

<120>  Novel applications of HIP/PAP or derivatives thereof

<130>  W224FR

<160>  3

<170>  PatentIn version 3.3

<210>  1
<211>  138
<212>  PRT
<213>  Homo sapiens

<400>  1

Ile Arg Cys Pro Lys Gly Ser Lys Ala Tyr Gly Ser His Cys Tyr Ala
1               5                   10                  15

Leu Phe Leu Ser Pro Lys Ser Trp Thr Asp Ala Asp Leu Ala Cys Gln
            20                  25                  30

Lys Arg Pro Ser Gly Asn Leu Val Ser Val Leu Ser Gly Ala Glu Gly
        35                  40                  45

Ser Phe Val Ser Ser Leu Val Lys Ser Ile Gly Asn Ser Tyr Ser Tyr
    50                  55                  60

Val Trp Ile Gly Leu His Asp Pro Thr Gln Gly Thr Glu Pro Asn Gly
65                  70                  75                  80

Glu Gly Trp Glu Trp Ser Ser Ser Asp Val Met Asn Tyr Phe Ala Trp
                85                  90                  95

Glu Arg Asn Pro Ser Thr Ile Ser Ser Pro Gly His Cys Ala Ser Leu
            100                 105                 110

Ser Arg Ser Thr Ala Phe Leu Arg Trp Lys Asp Tyr Asn Cys Asn Val
        115                 120                 125

Arg Leu Pro Tyr Val Cys Lys Phe Thr Asp
    130                 135

<210>  2
<211>  149
<212>  PRT
<213>  Homo sapiens

<400>  2

Glu Glu Pro Gln Arg Glu Leu Pro Ser Ala Arg Ile Arg Cys Pro Lys
1               5                   10                  15

Gly Ser Lys Ala Tyr Gly Ser His Cys Tyr Ala Leu Phe Leu Ser Pro
            20                  25                  30

Lys Ser Trp Thr Asp Ala Asp Leu Ala Cys Gln Lys Arg Pro Ser Gly
        35                  40                  45

Asn Leu Val Ser Val Leu Ser Gly Ala Glu Gly Ser Phe Val Ser Ser
    50                  55                  60

Leu Val Lys Ser Ile Gly Asn Ser Tyr Ser Tyr Val Trp Ile Gly Leu
65                  70                  75                  80

His Asp Pro Thr Gln Gly Thr Glu Pro Asn Gly Glu Gly Trp Glu Trp
                85                  90                  95

Ser Ser Ser Asp Val Met Asn Tyr Phe Ala Trp Glu Arg Asn Pro Ser
            100                 105                 110

Thr Ile Ser Ser Pro Gly His Cys Ala Ser Leu Ser Arg Ser Thr Ala
        115                 120                 125

Phe Leu Arg Trp Lys Asp Tyr Asn Cys Asn Val Arg Leu Pro Tyr Val
    130                 135                 140

Cys Lys Phe Thr Asp
145

<210>  3
<211>  150
<212>  PRT
<213>  Homo sapiens

<400>  3

Met Glu Glu Pro Gln Arg Glu Leu Pro Ser Ala Arg Ile Arg Cys Pro
1               5                   10                  15

Lys Gly Ser Lys Ala Tyr Gly Ser His Cys Tyr Ala Leu Phe Leu Ser
            20                  25                  30

Pro Lys Ser Trp Thr Asp Ala Asp Leu Ala Cys Gln Lys Arg Pro Ser
        35                  40                  45

Gly Asn Leu Val Ser Val Leu Ser Gly Ala Glu Gly Ser Phe Val Ser

    50                  55                  60

Ser Leu Val Lys Ser Ile Gly Asn Ser Tyr Ser Tyr Val Trp Ile Gly
65                  70                  75                  80

Leu His Asp Pro Thr Gln Gly Thr Glu Pro Asn Gly Glu Gly Trp Glu
                85                  90                  95

Trp Ser Ser Ser Asp Val Met Asn Tyr Phe Ala Trp Glu Arg Asn Pro
            100                 105                 110

Ser Thr Ile Ser Ser Pro Gly His Cys Ala Ser Leu Ser Arg Ser Thr
        115                 120                 125

Ala Phe Leu Arg Trp Lys Asp Tyr Asn Cys Asn Val Arg Leu Pro Tyr
    130                 135                 140

Val Cys Lys Phe Thr Asp
145                 150